# EUROPÄISCHE PATENTSCHRIFT

(11) **EP 2 536 441 B1**
(45) Veröffentlichungstag und Bekanntmachung des Hinweises auf die Patenterteilung: **08.07.2020**
(21) Anmeldenummer: 11705163.1
(22) Anmeldetag: 14.02.2011
(51) Int. Cl.: A61L 2/18, A61G 15/14

(54) **MEDIZINISCHES GERÄT, KONZENTRAT UND METHODE ZUR WASSERAUFBEREITUNG**
MEDICAL DEVICE, CONCENTRATE AND METHOD FOR TREATING WATER
APPAREIL MÉDICAL, CONCENTRÉ ET MÉTHODE DE TRAITEMENT DE L'EAU

(30) Priorität: 15.02.2010 AT 2212010
(43) Veröffentlichungstag der Anmeldung: 26.12.2012
(73) Patentinhaber: Pregenzer, Bruno, 6414 Mieming (AT); Geiger, Sebastian, 6521 Fliess (AT)
(72) Erfinder: Pregenzer, Bruno, 6414 Mieming (AT); Geiger, Sebastian, 6521 Fliess (AT)
(74) Vertreter: Laufhütte, Dieter
(86) Internationale Anmeldenummer: PCT/EP2011/000676
(87) Internationale Veröffentlichungsnummer: WO 2011/098294

(56) Entgegenhaltungen:
- EP-A1- 1 709 978
- WO-A1-03/064580
- WO-A2-98/40107
- DE-A1- 3 403 640
- US-B1- 6 448 062

## Beschreibung

Die Erfindung betrifft eine Behandlungseinheit für den zahnärztlichen Behandlungsplatz, die Verwendung eines wässrigen Konzentrats zur Beimengung zu in einer derartigen Behandlungseinheit benötigtem Wasser sowie ein Verfahren zur Aufbereitung des in einer derartigen Behandlungseinheit benötigten Wassers.

Im Stand der Technik spielt Wasserentkeimung in medizinischen Geräten, beispielsweise im Bereich der Zahnbehandlung und der Kieferchirurgie eine große Rolle um einer möglichen Keimübertragung durch eine wasserbasierte Behandlungsflüssigkeit vorzubeugen. Es sind mehrere Methoden bekannt, um das für eine zahnärztliche Behandlungseinheit verwendete Leitungswasser vor Ort aufzubereiten und zu desinfizieren. So offenbart die DE 34 03 640 C2 eine Vorrichtung zum Entkeimen einer wasserbasierten Behandlungsflüssigkeit durch Beimischung einer Desinfektionsflüssigkeit. Die DE 296 03 926 U1 offenbart die Keimtötung durch die Einwirkung von UV-Strahlung und zusätzlich die Zudosierung von Wasserstoffperoxid. Die DE 696 21 505 T2 betrifft eine Behandlungseinheit für den zahnärztlichen Behandlungsplatz mit einer Vorrichtung zur Zudosierung von Entkalkern und Desinfektionsmitteln.

In der Praxis weisen medizinische Geräte des Öfteren keine Mittel zur Desinfektion des Wassers auf. In diesem Fall erfolgt die Versorgung vielfach über isolierte Kanister, welche gesondert mit Wasser und einem Desinfektionsmittel befüllt wird. Diese Lösung bezeichnet man im Geschäftsverkehr auch als "Bottel-Lösung".

Die Wasserversorgung medizinischer Geräte unterliegt den Vorgaben des DVGW (Deutscher Verein des Gas- und Wasserfaches). Das entsprechende technische Regelwerk schreibt beispielsweise Maßnahmen vor, mit Hilfe derer der Rückfluss von mit biologischen Materialien kontaminiertem Wasser in die Wasserversorgung verhindert werden soll. Im Falle einer Versorgung aus einem Kanister entfällt diese Anforderung. Das über medizinische Geräte an den Patienten abgegebene Wasser unterliegt je nach Anwendung ferner zumindest den Anforderungen der Brauchwasserverordnung und die darin angegebenen Grenzwerte dürfen keinesfalls überschritten werden.

Neben einer Verwendung von keimfreiem Wasser ist, unter Einhaltung der Vorgaben des DVGW, jedoch insbesondere auch die Verwendung von enthärtetem Wasser interessant.

Im Stand der Technik ergeben sich durch die Verwendung von Wasser mit einem für Leitungswasser typischen Kalkgehalt in medizinischen Geräten immer wieder Probleme durch Kalkabscheidung. Diese tritt stärker, aber nicht ausschließlich an Stellen auf, wo wasserführende Leitungen erwärmt werden. Eine Erwärmung kann gewollt oder ungewollt durch elektrische Ventile, Heizblöcke und Motoren (beispielsweise Zahnbohrer) hervorgerufen werden. Aufgrund der Kalkablagerungen wird die Funktion der Gerätschaft gestört und zusätzliche Wartung oder Reparaturarbeiten sind notwendig.

Die Wasserenthärtung in der Versorgung von medizinischen Geräten stellt im Stand der Technik jedoch ein Problem dar. Es ist zwar möglich, in einer Praxis eine zentrale Entsalzungsanlage vorzusehen, welche Wasser z.B. durch den Einsatz von Ionenaustauschern oder reverser Osmose enthärten kann, jedoch ist diese Lösung nicht in jeder Hinsicht optimal. Während Ionenaustauscher leicht verkeimen, sind reverse Osmosegeräte verhältnismäßig teuer in der Anschaffung. In privaten Praxen, insbesondere privaten Dentalpraxen sind zentrale Entsalzungsanlagen daher wenig verbreitet. Auch das Entsalzen von Wasser unmittelbar vor oder innerhalb der medizinischen Geräte mit Ionenaustauschern oder reverser Osmose ist aus den oben genannten Gründen nur eingeschränkt möglich.

In der Praxis wird enthärtetes Wasser gegebenenfalls unter Verwendung einer Bottel-Lösung zur Versorgung von medizinischen Geräten genützt. Diese Lösung ist vergleichsweise praktisch und auch für private Praxen geeignet, jedoch ist die Anschaffung von und die Befüllung mit enthärtetem Wasser auch gewissermaßen aufwendig und mit Mehrkosten verbunden.

Ausgehend von der oben dargestellten Problematik soll durch die Erfindung die Kalkbildung in medizinischen Geräten verringert werden.

Ferner ist es eine Aufgabe der vorliegenden Erfindung, auf zentrale Entsalzungsanlagen verzichten zu können.

Des Weiteren soll eine einfache und billige Möglichkeit geschaffen werden, die Kalkabscheidung in einzelnen medizinischen Geräten oder Gerätegruppen individuell zu unterbinden.

Eines oder mehrere dieser Ziele werden mit einer Behandlungseinheit nach Anspruch 1 erreicht. Vorteilhafte Ausführungsformen ergeben sich aus den Unteransprüchen.

Demnach ist eine Behandlungseinheit für den zahnärztlichen Behandlungsplatz vorgesehen, welche an eine Wasserversorgung angeschlossen ist. Erfindungsgemäß soll die Behandlungseinheit Mittel zur Minderung der Kalkabscheidung und zur Entkeimung des durch die Wasserversorgung bereitgestellten Wassers aufweisen.

Durch den Zusatz von Komplexbildnern wird die Abscheidung von Kalk in Leitungen und anderen Teilen der erfindungsgemäßen Behandlungseinheit gemindert. Vorzugsweise wird die Kalkabscheidung vollständig oder im Wesentlichen vollständig verhindert.

Die "Wasserversorgung" kann ein zentraler, vorzugsweise DVGW-konformer Anschluss an das Wassernetz sein. In einer anderen Alternativ kann Wasser aus einem Behälter bezogen werden. In einer Ausführungsform wird nicht enthärtetes, calcium- bzw. magnesiumbicarbonathaltiges Wasser bereitgestellt.

In einer Ausführungsform weist die Behandlungseinheit Mittel zum Aufheizen zumindest eines Teils der Behandlungsflüssigkeit auf. Da die Abscheidung von Kalk bevorzugt an Stellen auftritt die erwärmt werden, ist die Enthärtung des zulaufenden Wassers in derartigen Behandlungseinheiten besonders vorteilhaft.

Zur Minderung der Kalkabscheidung weist die Behandlungseinheit zumindest einen Vorratsbehälter mit einem wässrigen Konzentrat enthaltend Komplexbildner und Desinfektionsmittel und eine Mischstelle auf. Die Mischstelle dient zur Beimengung des Konzentrats in durch die Wasserversorgung bereitgestelltes Wasser, beziehungsweise zur Einspritzung des Konzentrats in eine das Wasser führende Leitung.

Durch das Vorhandensein nur eines Vorratsbehälters und einer Mischstelle entsteht durch die Wasserenthärtung gegenüber vorbekannten Geräten kein zusätzlicher Aufwand. Ferner ist die Fehleranfälligkeit besonders gering.

Die Komplexierungsmittel umfassen einzelne Stoffe oder Kombinationen von Stoffen ausgewählt aus der Gruppe der der Polyphosphate, der 2-Phosphonobutan-1,2,4-tricarbonsäure (PBTC) und der niedermolekularen Polyacrylsäuren.

Das Komplexierungsmittel wird durch die Mittel in unterstöchiometrischen Mengen bezogen auf die Kalzium- bzw. Magnesiumionenkonzentration im Wasser zugegeben, da sich bei Verwendung von Polyphosphaten, PBTC und niedermolekolaren Polyacrylsäuren ein Threshold-Effekt einstellt und die Ausfällung von Kalk bereits durch unterstöchiometrische Zugabe verhindert wird.

Die Zugabe von unterstöchiometrischen Mengen an Komplexierungsmitteln ist vorteilhaft, da der Verbrauch an Komplexierungsmitteln geringer ist und Kosten gering gehalten werden. Ferner kann die Konzentration an Komplexierungsmitteln in der Behandlungsflüssigkeit gering gehalten werden wodurch die jeweiligen Auflagen einfacher erfüllbar sind.

Besonders bevorzugt ist PBTC, da sich bei dessen Verwendung ein Treshold-Effekt einstellt und die gebildeten Komplexe unter den gewünschten Bedingungen stabil sind.

In einer weiteren, bevorzugten Ausführungsform werden Desinfektionsmittel aus der Gruppe der anorganischen und organischen Peroxide, der Metallionen, beispielsweise Silber- und/oder Kupferionen, der Alkohole, der Aldehyde, der organischen und anorganischen Chlorverbindungen oder Chlorabspalter wie beispielsweise Chloramin-T, des Guanidins, der Biguanidine, der organisch modifizierten Biguanidine, wie beispielsweise PHMBG (Polyhexamethylenbiguanidin) oder Chlorhexidin, und der quaternären Ammoniumverbindungen (kurz: QUATs) ausgewählt. In einer besonders bevorzugten Ausführungsform ist das Desinfektionsmittel Wasserstoffperoxid.

Es hat sich gezeigt, dass durch die Kombination von Desinfektionsmitteln und Komplexierungsmitteln keine Verschlechterung der keimreduzierenden Eigenschaften bezogen auf die alleinige Zugabe von Desinfektionsmitteln beobachtet wird. Mit anderen Worten beeinflussen sich die Funktion des Desinfektionsmittels und des Komplexierungsmittels bei geeigneter Kombination, beispielsweise Wasserstoffperoxid und PBTC, nicht.

In einer weiteren Ausführungsform ist vorgesehen, dass die Behandlungseinheit neben dem Zusatz von Komplexbildnern keine weiteren Mittel zur Minderung der Kalkabscheidung oder der Wasserenthärtung aufweist. Demgemäß kann auf Enthärtungsvorrichtungen aus dem Stand der Technik, beispielsweise wie Ionenaustauscher oder reverse Osmosegeräte, verzichtet werden und Installations- bzw. Unterhaltskosten eingespart werden.

Die Erfindung betrifft ferner die Verwendung eines wie beschriebenen wässrigen Konzentrats, welches sich zur Zudosierung zu in einer erfindungsgemäßen Behandlungseinheit benötigtem Wasser eignet und/oder dafür bestimmt ist. Das Konzentrat weist die beschriebenen Komplexierungsmittel auf und mindert oder verhindert die Kalkabscheidung aus nicht enthärtetem Wasser.

Letztlich betrifft die Erfindung ein Verfahren zur Aufbereitung von in einer erfindungsgemäßen Behandlungseinheit benötigtem Wasser, wobei die Aufbereitung die Kalkabscheidung minimierend und ferner keiminhibierend wirken soll. Das Verfahren zeichnet sich erfindungsgemäß durch die Zugabe eines wie oben beschriebenen Konzentrats aus.

Weitere Einzelheiten und Vorteile ergeben sich aus dem nachstehenden Ausführungsbeispiel.

Ein zahnärztlicher Behandlungsplatz mit verschiedenen Behandlungsgeräten wird für die Demonstration der Vorteile eines erfindungsgemäßen Geräts verwendet. Der Behandlungsplatz umfasst eine Behandlungsvorrichtung mit einem Speibecken, einer Speifontäne, einem Hahn für Mundspülwasser, einer Versorgungsleitung für Kühlwasser und Wasserversorgungsleitungen für austauschbare Handwerkzeuge einer zahnärztlichen Behandlungseinheit, wie z.B. Bohrer oder Spüldüsen. Der Behandlungsplatz ist den Vorgaben der DVGW entsprechend an eine Wasserleitung angeschlossen, welche normales Leitungswasser in Trinkwasserqualität liefert. Die Behandlungseinrichtung weist eine im Stand der Technik übliche Vorrichtung zum Entkeimen des zugeführten Wassers auf, wobei ein H₂O₂ - Konzentrat aus einem Vorratsbehälter zum aus der Versorgungsleitung kommenden Wasser beigemengt wird.

Zur Erprobung der Erfindung wird das Gerät komplett entkalkt und das H₂O₂-Konzentrat mit einem Konzentrat bestehend aus einer konzentrierten, wässrigen Lösung von H₂O₂ und 2-Phosphonobutan-1,2,4-tricarbonsäure (PBTC) ersetzt. Die kontinuierliche Zudosierung zur wasserführenden Leitung und das Konzentrationsverhältnis zwischen H₂O₂ und PBTC wird so eingestellt, dass Wasserstoffperoxid in derselben Menge wie zuvor, und PBTC im Verhältnis zur Gesamtwasserhärte in leicht unterstöchiometrischer Menge zudosiert wird.

In Wasserproben wurde festgestellt, dass durch die Kombination von H₂O₂ und PBTC keine Verschlechterung der keimreduzierenden Eigenschaften bezogen auf H₂O₂ ohne PBTC eingetreten ist, und dass die Vorgaben der Brauchwasserverordnung erfüllt sind.

Nach sechsmonatiger Anwendung im täglichen Zahnarztgebrauch werden bisher an den Behandlungsgeräten beobachtete Kalkablagerungen nicht mehr beobachtet. Beim Unterhalt der Behandlungsvorrichtung entstanden abgesehen vom Austausch der Konzentrate keinerlei Zusatzkosten.

## Patentansprüche

1. Behandlungseinheit für den zahnärztlichen Behandlungsplatz, welche an eine Wasserversorgung angeschlossen ist,
wobei die Behandlungseinheit Mittel zur Minderung der Kalkabscheidung aus und zum Entkeimen von durch die Wasserversorgung bereitgestelltem Wasser aufweist,
wobei diese Mittel zumindest einen Vorratsbehälter mit einem wässrigen Konzentrat enthaltend Komplexbildner und Desinfektionsmittel und eine Mischstelle zur Beimengung des Konzentrats in durch die Wasserversorgung bereitgestelltes Wasser umfassen, und wobei der Komplexbildner aus der Gruppe Polyphosphate, 2-Phosphonobutan-1,2,4-tricarbonsäure (PBTC) und niedermolekulare Polyacrylsäuren ausgewählt sind, und wobei die Mittel ausgebildet sind, um die Komplexbildner in unterstöchiometrischen Mengen zuzugeben.

2. Behandlungseinheit nach Anspruch 1, wobei das Desinfektionsmittel ein oder mehrere anorganische und/oder organische Peroxide, insbesondere Wasserstoffperoxid, Metallionen, insbesondere Silberionen und Kupferionen, Alkohole, Aldehyde, anorganische und organische Chlorverbindungen und/oder Chlorabspalter, gegebenenfalls Chloramin-T, Guanidin, Biguanidine, insbesondere organisch modifizierte Biguanidine und/oder quaternäre Ammoniumverbindungen (QUAT) oder Kombinationen daraus umfasst.

3. Verwendung eines wässrigen Konzentrats zur Beimengung zu in einer Behandlungseinheit nach einem der Ansprüche 1 bis 2 benötigtem Wasser, wobei das Konzentrat ein oder mehrere Komplexbildner aus der Gruppe Polyphosphate, 2-Phosphonobutan-1,2,4-tricarbonsäure (PBTC) und niedermolekulare Polyacrylsäuren und ein oder mehrere Desinfektionsmittel aufweist, und wobei die Komplexbildner geeignet sind, die Kalkabscheidung aus nicht enthärtetem Wasser bei unterstöchiometrischer Zugabe zu mindern oder zu verhindern.

4. Verfahren zur Aufbereitung von in einer Behandlungseinheit nach einem der Ansprüche 1 bis 2 benötigten Wassers, wobei die Aufbereitung die Kalkabscheidung mindernd und ferner keiminhibierend wirkt, aufweisend den Schritt der Zugabe eines Konzentrats, das ein Komplexierungsmittel aus der Gruppe Polyphosphate, 2-Phosphonobutan-1,2,4-tricarbonsäure (PBTC) und niedermolekulare Polyacrylsäuren und ein Desinfektionsmittel aufweist, wobei die Zugabe bezogen auf die Menge des Komplexierungsmittels in unterstöchiometrischer Menge erfolgt.

5. Verfahren nach Anspruch 4, wobei das Gerät mit nicht enthärtetem, bicarbonathaltigem Wasser versorgt wird.

## Claims

1. Treatment unit for the dental treatment station, which is connected to a water supply,
wherein the treatment unit comprises means for reducing lime deposits from and for sterilizing water provided by the water supply,
wherein said means comprise at least one storage container with an aqueous concentrate containing complexing agents and disinfectants and an admixing location for admixing the concentrate to water provided by the water supply, and wherein the complexing agent is selected from the group polyphosphates, 2-Phosphonobutane-1,2,4-tricarboxylic acid (PBTC) and low-molecular polyacrylic acids, and wherein the means are configured for adding the complexing agents in a sub-stoichiometric manner.

2. Treatment unit according to claim 1, wherein the disinfectant comprises one or multiple inorganic and/or organic peroxides, in particular hydrogen peroxide, metal ions, in particular silver ions and copper ions, alcohols, aldehydes, inorganic and organic chlorine compounds and/or chlorine splitting agents, possibly chloramine T, guanidine, biguanides, in particular organically modified biguanides and/or quaternary ammonia compounds (QUAT) or combinations thereof.

3. Use of an aqueous concentrate for admixing to water required in a treatment unit in accordance with one of claims 1 to 2, wherein the concentrate comprises one or multiple complexing agents from the group polyphosphates, 2-Phosphonobutane-1,2,4-tricarboxylic acid (PBTC) and low-molecular polyacrylic acids, and one or more disinfectants, and wherein the complexing agents are suitable to reduce or prevent the deposition of lime from non-softened water by being admixed in a sub-stoichiometric manner.

4. Method for treating water required in a treatment unit in accordance with any one of claims 1 to 2, wherein the treatment reduces the lime deposition and furthermore acts in a germicidal manner, comprising the step of admixing a concentrate which comprises a complexing agent from the group polyphosphates, 2-Phosphonobutane-1,2,4-tricarboxylic acid (PBTC) and low-molecular polyacrylic acids, and a disinfectant, wherein the admixing occurs in a sub-stoichiometric amount with respect to the amount of complexing agent.

5. Method according to claim 4, wherein the apparatus is supplied with non-softened water that contains bicarbonate.

## Revendications

1. Unité de traitement pour poste de soins dentaires, qui est raccordée à une alimentation en eau,
l'unité de traitement comportant des moyens destinés à réduire le dépôt de calcaire provenant d'eau fournie par l'alimentation en eau et stériliser celle-ci,
ces moyens comprenant au moins un réservoir avec un concentré aqueux contenant des agents complexants et des désinfectants et un point de mélange pour ajouter le concentré dans de l'eau fournie par l'alimentation en eau, et les agents complexants étant choisis dans le groupe des polyphosphates, de l'acide 2-phosphonobutane-1,2,4-tricarboxylique (PBTC) et des acides polyacryliques de faible poids moléculaire, et les moyens étant conçus pour ajouter les agents complexants en quantités sous-stœchiométriques.

2. Unité de traitement selon la revendication 1, dans laquelle le désinfectant comprend un ou plusieurs peroxydes inorganiques et/ou organiques, en particulier du peroxyde d'hydrogène, des ions métalliques, en particulier des ions d'argent et des ions de cuivre, des alcools, des aldéhydes, des composés chlorés et/ou déchlorinateurs inorganiques et organiques, le cas échéant de la chloramine-T, de la guanidine, des biguanides, en particulier des biguanides organiquement modifiées et/ou des composés d'ammonium quaternaires (CAQ) ou des combinaisons de ceux-ci.

3. Utilisation d'un concentré aqueux destiné à être ajouté à de l'eau requise dans une unité de traitement selon la revendication 1 ou 2, le concentré comportant un ou plusieurs agents complexants du groupe des polyphosphates, de l'acide 2-phosphonobutane-1,2,4-tricarboxylique (PBTC) et des acides polyacryliques de faible poids moléculaire et un ou plusieurs désinfectants, et les agents complexants étant adaptés pour réduire ou empêcher le dépôt de calcaire d'eau non adoucie par ajout sous-stœchiométrique.

4. Procédé de traitement d'eau requise dans une unité de traitement selon la revendication 1 ou 2, le traitement ayant une action de réduction du dépôt de calcaire ainsi que d'inhibition des germes, comportant l'étape d'ajout d'un concentré, qui comporte un agent complexant du groupe des polyphosphates, de l'acide 2-phosphonobutane-1,2-4-tricarboxylique (PBTC) et des acides polyacryliques de faible poids moléculaire et un désinfectant, l'ajout étant effectué, en ce qui concerne la quantité de l'agent complexant, en quantité sous-stœchiométrique.

5. Procédé selon la revendication 4, dans lequel l'appareil est alimenté en eau non adoucie contenant du bicarbonate.
